# EUROPÄISCHE PATENTSCHRIFT

(11) **EP 0 460 467 B1**
(45) Veröffentlichungstag und Bekanntmachung des Hinweises auf die Patenterteilung: **23.08.1995**
(21) Anmeldenummer: 91108437.4
(22) Anmeldetag: 24.05.1991
(51) Int. Cl.: A61F 5/44, A61F 13/15

(54) **Hosenartige Wegwerfkleider**
Pants-type disposable clothing
Vêtements jetables sous forme de culottes

(30) Priorität: 24.05.1990 JP 134439/90
(43) Veröffentlichungstag der Anmeldung: 11.12.1991
(73) Patentinhaber: UNI-CHARM CORPORATION, Kawanoe-shi Ehime-ken (JP)
(72) Erfinder: Suzuki, Migaku, Kamakura-shi, Kanagawa-ken (JP); Kudo, Takeshi, Kawanoe-shi, Ehime-ken (JP); Sakai, Ritsuko, Kakegawa-shi, Shizuoka-ken (JP); Yamaki, Rumi, Kakegawa-shi, Shizuoka-ken (JP)
(74) Vertreter: Sperling, Rüdiger, Dipl.-Ing.

(56) Entgegenhaltungen:
- EP-A- 0 164 435
- CH-A- 282 061
- US-A- 2 977 957
- US-A- 4 771 483

## Beschreibung

Die vorliegende Erfindung betrifft Wegwerfkleider und insbesondere solche Kleider, die hosenartig und in einer vereinfachten Form sind, gemäß dem Oberbegriff des Anspruchs 1.

Obwohl die meisten der üblicherweise verwendeten Wegwerfkleider wie Windeln, Trainingshosen und Inkontinenzslips vom sogenannten offenen Typ sind, wurden solche Wegwerfkleider auch beispielsweise im U.S. Patent Nr. 4,771,483 vorgeschlagen.

Die vorliegende Erfindung betrifft hosenartige Wegwerfkleider mit einer vereinfachten Form. Das oben genannte U.S. Patent Nr. 4,771,483 liegt dem Oberbegriff des Anspruchs 1 zugrunden. schlägt ebenfalls solche hosenartigen Wegwerfkleider in vereinfachter Form vor. Gemäß diesem Stand der Technik wird ein rechtwinkeliges Schichtmaterial entlang jedem der gegenüberliegenden Seitenränder vorgesehen, das einen einzelnen Schlitz aufweist, der im wesentlichen parallel zu dem zugehörigen der gegenüberliegenden Seitenränder verläuft. Ein schmaler Bereich, der zwischen dem Schlitz und dem zugehörigen der gegenüberliegenden Seitenränder definiert ist, wird mit einem geeigneten elastischen Teil unter dessen gestrecktem Zustand eingefügt, um ein Paar von die Taillenöffnung umgebenden elastischen Bändern zu bilden. Gemäß diesem Stand der Technik gibt es jedoch im wesentlichen keine Maßnahmen, ein Auslaufen von Körperflüssigkeit zu vermeiden, das möglicherweise um die Beinöffnungen erfolgt, was für die Wegwerfkleider im allgemeinen kritisch ist und deshalb ein Problem bei der praktischen Anwendung ergeben kann. In Anbetracht einer solchen Situation ist es Aufgabe der vorliegenden Erfindung, neue hosenartige Wegwerfkleider bereitzustellen, die eine wirksame Vermeidung des Auslaufens von Körperflüssigkeit in Verbindung mit einer einfachen Form ermöglichen.

Die Erfindung umfaßt ein hosenartiges Wegwerfkleid, umfassend eine Grundanordnung, die mindestens eine flüssigkeitsdurchlässige Oberschicht und eine flüssigkeitsundurchlässige rückwärtige Schicht enthält, u. mit elastischen Bändern, die mittels Befestigungslinien auf der Grundanordnung angebracht sind, um eine Taillenöffnung teilweise zu umgeben, wobei die Grundanordnung Umrißlinien hat, die jeweils längs verlaufende, gegenüberliegende Seitenränder und quer verlaufende, gegenüberliegende Endränder enthalten, dadurch gekennzeichnet, daß jeder der gegenüberliegenden Seitenränder mit einem Bund versehen ist, der sich entlang des Längsverlaufenden Seitenrands erstreckt und im Zusammenwirken mit dem elastischen Band jeweils eine Beinöffnung umgibt, wobei ein äußerer Seitenrand des die Beinöffnung umgebenden Bundes entlang dem zugehörigen gegenüberliegenden Seitenrand der Grundanordnung angebracht ist, und jeweilige Endränder des die Beinöffnung umgebenden Bundes auf der Grundanordnung mittels jeweiliger Befestigungslinien angebracht sind, die an den Umrißlinien des Kleids vorgesehen sind. Diese und die anderen Merkmale der Erfindung werden durch die folgende Beschreibung besser veranschaulicht.

Gemäß vorliegender Erfindung wird ein Paar Bünde (flaps) vorgesehen, welche die Beinöffnung umgeben und längs der Kleidungsstücke, jeweils entlang deren gegenüberliegenden Seitenrändern verlaufen. Bei jedem Bund, der die Beinöffnung umgibt, ist der äußere Seitenrand flüssigkeitsdicht am zugehörigen gegenüberliegenden Seitenrand der Kleidungsstücke befestigt, und ihr innerer Seitenrand wirkt mit der Oberschicht der Kleidungsstücke zusammen, um eine Tasche zu bilden, die hin zu einer Mittellinie geöffnet ist, die sich längs des Kleidungsstücks erstreckt. Die Tasche ist bei der Verhinderung eines Auslaufens von Körperausscheidungen wirksam, das ansonsten um die Beine des Trägers herum auftreten könnte. Weiterhin ist ein Paar von elastischen Bändern vorgesehen, welche die Taillenöffnung umgeben und sich entlang der gegenüberliegenden Seitenränder erstrecken. Diese elastischen Bänder werden auf der Grundanordnung der Kleidungsstücke nur an deren Endrändern entlang den jeweiligen Umrißlinien der Grundanordnung befestigt. Demgemäß sind die Kleider hervorragend in ihrer Funktion und sehr einfach in der Form, wodurch ermöglicht wird, daß sie leicht zusammengesetzt und den Benutzern mit annehmbar niedrigem Preis angeboten werden können.

Es zeigt:
Figur 1 eine perspektivische Ansicht, die eine hosenartige Wegwerfwindel als eine Ausführungsform der vorliegenden Erfindung zeigt;
Figur 2 eine Draufsicht derselben;
Figur 2A bis 2C fragmentarische Draufsichten, die beispielhaft zeigen, wie der Endrand entlang dem Umriß der Kleidungsstücke befestigt ist;
Figur 3A bis 3C Teilschnitte, die entlang einer Linie Y-Y in Figur 2 verlaufen und beispielhaft die Mittel der Randbefestigung verdeutlichen;
Figur 4 eine perspektivische Ansicht, die im Teilschnitt ein Beispiel für die überlappende Befestigung des Endrands zeigt;
Figur 5 und 6 eine perspektivische Ansicht bzw. eine perspektivische Ansicht im Teilschnitt, die entlang einer Linie Z-Z in Figur 5 verläuft, und eine andere Ausführungsform der Erfindung zeigt;
Figur 7 und 8 eine perspektivische Ansicht und eine fragmentarische Draufsicht, die noch eine weitere Ausführungsform der Erfindung zeigen.

Die Erfindung wird unter Verweis auf die beigefügten Zeichnungen detaillierter beschrieben. Es sollte klar sein, daß jede Wegwerfwindel der Ausführungsform im Hinblick auf eine darin längs verlaufende Mittellinie seitensymmetrisch ist.

### [Ausführungsform 1]

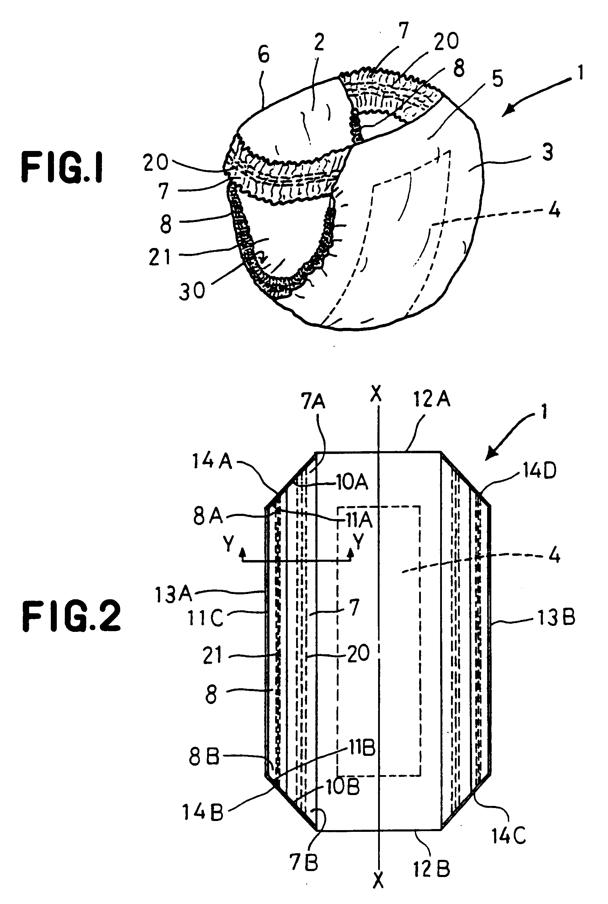

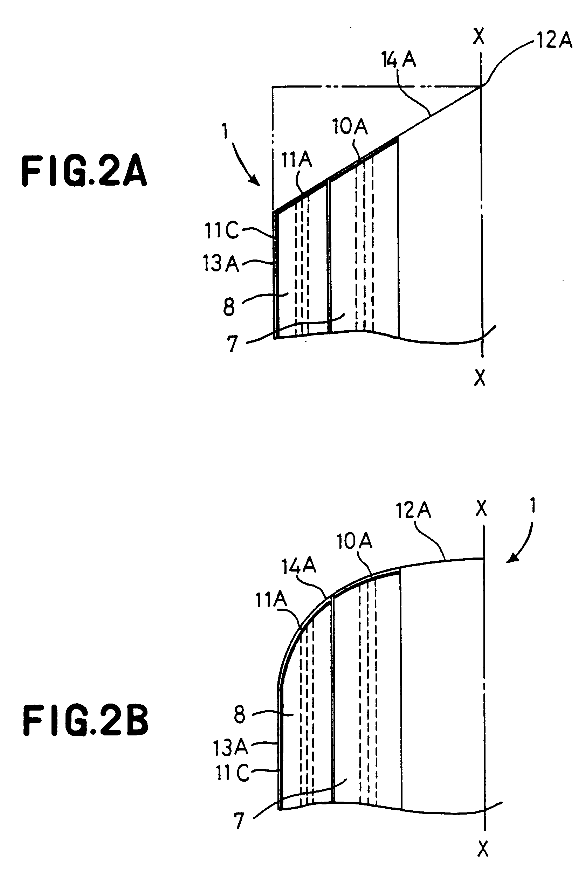

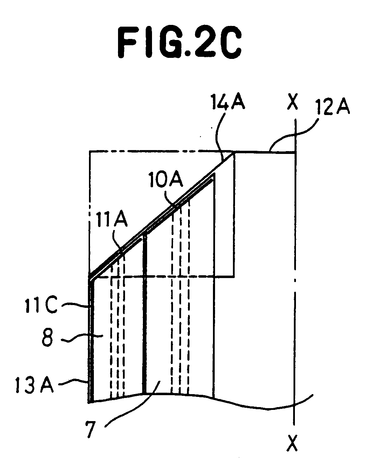

Figur 1 ist eine perspektivische Ansicht einer sogenannten hosenartigen Wegwerfwindel 1, die als spezifische Ausführungsform der Erfindung hergestellt worden ist, und Figur 2 ist eine Draufsicht auf dieselbe Windel 1, die längsgestreckt ist und in eine flache Lage gebracht ist, wobei ihre Innenseite nach oben zeigt. Da die Windel 1, wie zuvor erwähnt, hinsichtlich der längs verlaufenden Mittellinie seiten-symmetrisch ist, soll nur die linke Hälfte der Windel der Ansicht aus Figur 2 detailliert beschrieben werden.

In Figur 1 enthält die Windel 1 eine Grundanordnung, die eine wasserdurchlässige Oberschicht 2, eine wasserundurchlässige rückwärtige Schicht 3 und einen dazwischen angeordneten Absorptionskern 4. Ein Paar von elastischen Bändern 7, welche die Taillenöffnung umgeben und einen Vorderkörper 5 mit einem rückwärtigen Körper 6 in der Grundanordnung verbinden, und ein Paar von Beinöffnungen umgebenden Bünden 8 sind auf der Grundanordnung festgelegt. Jedes der elastischen Bänder 7, welche die Taillenöffnung umgeben, hat eine Vielzahl von Gummistreifen 20 und jeder Bund, der eine Beinöffnung umgibt, hat auch eine Vielzahl von Gummistreifen 21, die beide in Form von elastischen Zügen hergestellt sind. Sowohl das die Taillenöffnung umgebende elastische Band 7 als auch der die Beinöffnung umgebende Bund 8 enthalten wasserundurchlässige Materialien und vorzugsweise luftdurchlässige, aber wasserundurchlässige Materialien, wie etwa nichtgewobener Stoff, der aus geeigneten hydrophoben Fasern hergestellt ist.

Aus Figur 2 ist ersichtlich, daß sich die die Beinöffnung umgebenden schmalen Bünde 8 jeweils entlang gegenüberliegender Seitenränder der Windel 1 erstrecken, wobei jeder der Bünde an seinem äußeren Seitenrand an der Oberschicht 2 oder an der rückwärtigen Schicht 3 der Grundanordnung entlang einer Verbindungslinie 11C befestigt ist und der innere Seitenrand dem äußeren Seitenrand des zugehörigen, die Taillenöffnung umgebenden elastischen Band 7 gegenüberliegt. Die längs verlaufenden, gegenüberliegenden Enden 8A, 8B des Bundes 8 sind an der Oberschicht 2 oder an der rückwärtigen Schicht 3 jeweils entlang von Befestigungslinien 11A, 11B befestigt und entsprechend sind die längs verlaufenden, gegenüberliegenden Enden 7A, 7B des elastischen Bandes daran jeweils entlang von Befestigungslinien 10A, 10B befestigt, wobei die Befestigungslinien 11A, 10A und 11B, 10B jeweils entlang den Umrißlinien 14A, 14B der Grundanordnung verlaufen, wie später noch detaillierter beschrieben wird. Die Befestigungslinien 10A, 10B, 11A, 11B und 11C werden durch bekannte Mittel wie etwa Adhäsion und Heißschmelzschweißen gebildet, wobei jeweils mindestens die Linien 11A bis 11C durchgehend sein müssen, um ein Auslaufen von Körperausscheidungen zu verhindern, das möglicherweise um die Beine des Benutzers herum auftritt. Somit ist eine Tasche 30 zusammenwirkend definiert, die hin zur Mittellinie X-X geöffnet ist. Zusätzlich ist es bevorzugt, daß die jeweiligen benachbarten Linien 10A, 11A und 10B, 11B durchgehend in jeweilige Einzellinien sind, um ein gutes Aussehen des um diese Linien befindlichen Bereichs zu erreichen.

Die Linien 10A, 11A sind entlang einer geraden Linie 14A vorgesehen, die einer Umrißlinie entspricht, welche einen längs verlaufenden Endrand 12A und einen Seitenrand 13A der Grundanordnung schneidet, während die Linien 10B, 11B entlang einer geraden Linie 14B vorgesehen sind, die einem Umrißbereich entspricht, der den anderen Endrand 12B und den Seitenrand 13A schneidet. Auf diese Weise ist das die Taillenöffnung umgebende elastische Band 7 eingerichtet, einen natürlichen Sitz um die zugehörige Seite der Taille des Trägers (nicht gezeigt) zu haben, wie aus Figur 1 hervorgeht.

In Figur 2A ist eine andere Ausführungsform der oben erwähten Befestigung gezeigt, bei der die gerade Linie 14A einer Um-rißlinie entspricht, die einen Schnittpunkt des Endrands 12A und der längs verlaufenden Mittellinie X-X mit dem Seitenrand 13A verbindet. In dieser Ausführungsform sind die gegenüberliegenden Endränder der Grundanordnung im wesentlichen durch die Punkte 12A bzw. 12B definiert, und auch eine solche Konfiguration wird vom Umfang der vorliegenden Erfindung erfaßt.

Figur 2B zeigt noch eine weitere Ausführungsform der oben erwähnten Befestigung, bei der die gerade Linie 14A, die der Umrißlinie entspricht, durch eine gekrümmte Linie ersetzt ist. Auch eine solche Konfiguration ist vom Bereich der vorliegenden Erfindung abgedeckt.

Figur 2C zeigt einen weiteren Fall, bei dem die gerade Linie 14A, die der Umrißlinie entspricht, durch Faltung eines Eckbereichs zurück nach innen gebildet wird, der zwischen dem Endrand 12A und dem Seitenrand 13A definiert ist. In diesem Fall kann das Oberblatt 2 in Flächen-an-Flächen-Beziehung mittels Adhäsion befestigt werden, um jede unerwünschte Verschiebung davon zu vermeiden. Das die Taillenöffnung umgebende elastische Band 7 und der die Beinöffnung umgebende Bund 8 sind jeweils an der rückwärtigen Schicht 3 am gefalteten Eckbereich durch die Befestigungslinien 10A, 11A befestigt, die entlang der geraden Linie 14A verlaufen.

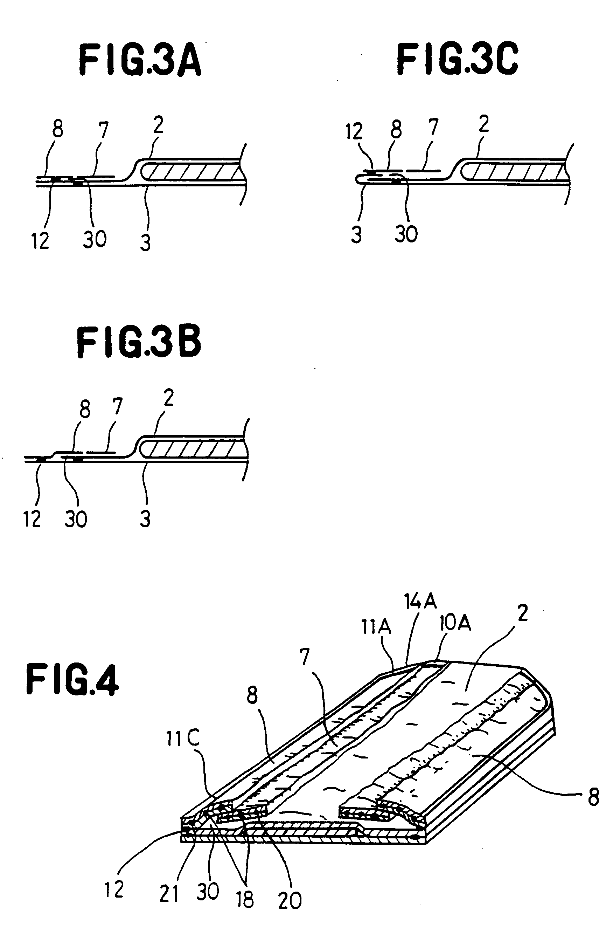

Figuren 3A bis 3C und Figuren 4A und 4B zeigen verschiedene weitere Variationen der obigen Befestigung. Figuren 3A, 3B und 3C zeigen schematisch in einem Schnitt, der zwischen den Pfeilen Y-Y in Figur 2 verläuft und in Richtung dieser Pfeile betrachtet wird, wie der Bund 8 entlang seines Seitenrandes an der Grundanordnung der Windel 1 befestigt ist. Figur 3A zeigt spezifisch einen Fall, bei dem der Bund 8 an die Oberschicht 2 mittels eines Heißschmelz-Klebstoffs 12 befestigt ist, Figur 3B zeigt einen Fall, bei dem der Bund 8 an der rückwärtigen Schicht 3 befestigt ist, die sich vom äußeren Seitenrand der Oberschicht 2 nach außen erstreckt, und Figur 3C zeigt einen Fall, bei dem die rückwärtige Schicht 3, die sich vom Seitenrand der Oberschicht 2 in Figur 3B nach außen erstreckt, zurück nach innen gefaltet ist und der Bund 8 über diesem gefalteten Rand befestigt ist. Die Methoden zur Befestigung, wie sie in Figur 3B und 3C gezeigt werden, sind vorteilhaft, da eine Körperausscheidung, welche die wasserdurchlässige Oberschicht 2 durchdrungen hat, innerhalb der Tasche 30 eingedämmt wird und wirksam an einem Auslaufen durch die Seitenränder der Windel 1 gehindert wird.

Die Befestigung kann, wie oben beschrieben, entlang von gegenüberliegenden Seitenrändern und den jeweiligen Umrißlinien der Grundanordnung erfolgen, um die in Figur 2 dargestellte Windel 1 herzustellen. Die Gummistreifen 20, die zu dem die Taillenöffnung umgebenden elastischen Band 7 gehören, und die Gummistreifen 21, die zu dem die Beinöffnung umgeben-den Bund 8 gehören, sind jeweils zuvor in gestrecktem Zustand an dem elastischen Band 7 bzw. dem Bund 8 angebracht worden, so daß, wenn auf die Windel 1 keine äußere Kraft ausgeübt wird, sich die jeweiligen Gumminstreifen kontrahieren, um Züge im elastischen Band 7 und dem Bund 8 zu bilden, während der innere Seitenrand des Bunds 8 mehr oder weniger zum Anheben und zur Bildung der Tasche 30 im Zusammenwirken mit der Oberschicht 2 neigt.

Obwohl diese spezifische Ausführungsform einschließlich des Absorptionskerns 4 gezeigt wurde, ist der Absorptionskern 4 für die vorliegende Erfindung nicht essentiell.

### [Ausführungsform 2]

Figur 4 zeigt teilweise im Querschnitt eine andere Ausführungsform der Erfindung. Diese Ausführungsform ist der Ausführungsform 1 ähnlich, abgesehen davon, daß das die Taillenöffnung umgebende elastische Band den zugehörigen, die Beinöffnung umgebenden Bund überlappt, so daß das elastische Band 7 sich teilweise zwischen dem Bund 8 und der Oberschicht 2 befindet. In dieser Ausführungsform erstrecken sich sowohl die Befestigungslinie 10A für das elastische Band 7 als auch die Befestigungslinie 11A für den Bund 8 entlang der geraden Linie 14A, die einer Umrißlinie der Grundanordnung entspricht, und definieren im wesentlichen zusammen eine durchgehende Befestigungslinie. Bei einer solchen Anordnung hebt das elastische Band 7, das sich unterhalb des Bundes 8 befindet, um die Linien 10A, 11A den Bund 8 von der Oberschicht 2 weg, wenn ein Benutzer beim Tragen der Windel 1 das elastische Band 7 und den zugehörigen Bund 8 weit trennt, wodurch die Tasche 30 weit geöffnet wird.

Wie aus Figur 4 klar ersichtlich ist, sind die Gummistreifen 20, 21 jeweils mit Trägerschichten bedeckt, so daß die Haut eines Trägers gegen direkten Kontakt mit diesen Gummistreifen 20, 21 geschützt ist.

### [Ausführungsform 3]

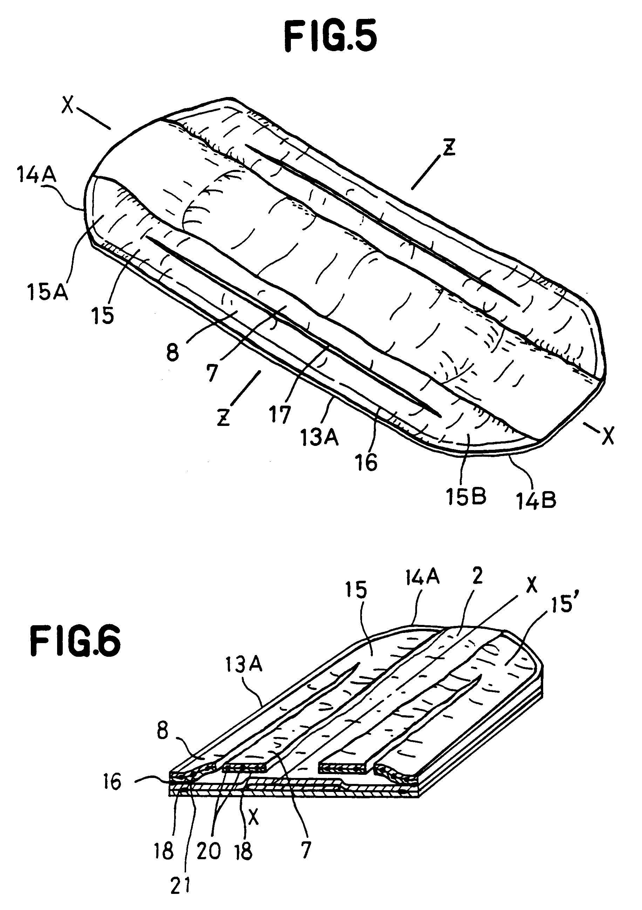

Figur 5 zeigt eine spezifische Ausführungsform, bei der das die Taillenöffnung umgebende Band 7 und der zugehörige, die Beinöffnung umgebende Bund, miteinander eine Einheit bilden. Ein wasserundurchlässiges, vorzugsweise luftdurchlässiges, aber wasserundurchlässiges Schichtteil 15 ist an der Oberschicht 2 entlang von jedem seiner gegenüberliegenden Seitenränder durch eine Befestigungslinie 16 befestigt, die aus heißschmelzendem Klebstoff 12 hergestellt ist, und diese Befestigungslinie 16 verläuft zu den gegenüberliegenden Endrändern der Windel 1, um gegenüberliegende Ränder 15A, 15B des Schichtteils 15 ebenfalls an der Oberschicht 2 zu befestigen. Das Schichtteil 15 ist mit einem, längs darauf verlaufenden Schlitz 17 versehen, so daß der Schlitz 17 das Schichtteil 15 in das die Taillenöffnung umgebende elastische Band 7 auf der Seite der Mittellinie X-X, die längs der Windel 1 verläuft, und in den die Beinöffnung umgebenden Bund 8 auf der Seite des Seitenrandes 13A teilt, wobei das elastische Band 7 und der Bund 8 an den gegenüberliegenden Enden des Schlitzes 17 miteinander eine Einheit bilden.

Figur 6 zeigt einen entlang einer Linie Z-Z in Figur 5 verlaufenden Schnitt. Wie zu sehen ist, wird das elastische Band 7 mit den Gummistreifen 20 und der Bund 8 mit den Gummistreifen 21 eingefügt. Für die vorliegende Erfindung ist es nicht kritisch, ob die Gummistreifen 20, 21 in ihrem Material, ihrer Konfiguration, Anzahl oder Menge, ihrem Ausdehnungswert und anderen Faktoren gleich sind oder nicht.

### [Ausführungsform 4]

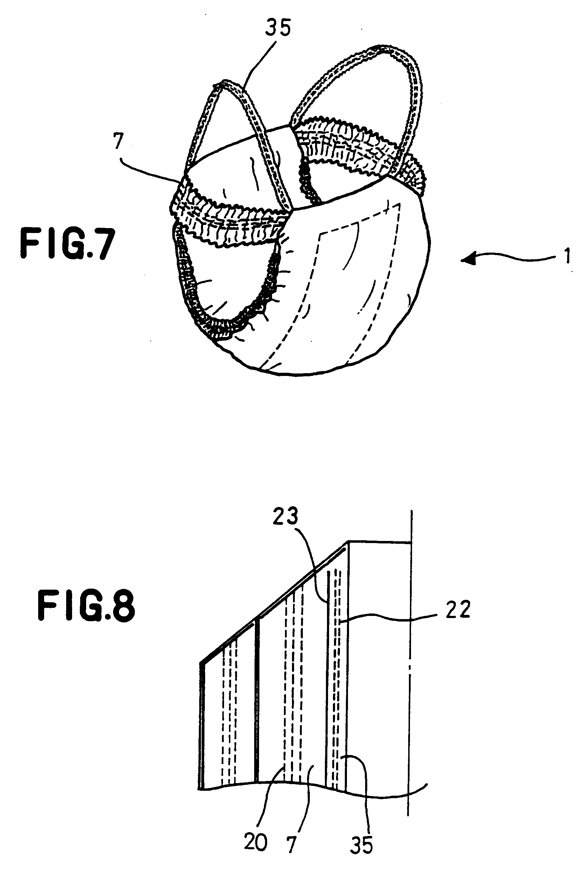

Figur 7 zeigt einen Fall, bei dem jedes der elastischen Bänder 7 der Taillenöffnung einen Schulterriemen 35 bildet.

Figur 8 zeigt die Windel 1, die gestreckt und in ihre flache Lage gebracht worden ist. Wie aus Figur 8 hervorgeht, ist das elastische Band 7 der Taillenöffnung mit einem Längsschlitz 23 versehen, so daß ein schmaler Teil des elastischen Bandes 7, der zwischen seinem inneren Seitenrand und dem Schlitz 23 definiert ist, als Schulterriemen 35 verwendet werden kann. Der Schulterriemen 35 kann, sofern erwünscht, mit einer geeigneten Anzahl von Gummistreifen 22, wie durch unterbrochene Linien gezeigt, versehen werden, und diese Gummistreifen 22 können zur gleichen Zeit am Schulterriemen 35 befestigt werden, wenn die Befestigung der Gummistreifen 20 an dem elastischen Band 7 erfolgt. Es ist für die Erfindung nicht kritisch, ob die Gummistreifen 20, 22 ähnlich sind oder nicht.

## Patentansprüche

1. Hosenartiges Wegwerfkleid, umfassend eine Grundanordnung, die mindestens eine flüssigkeitsdurchlässige Oberschicht (2) und eine flüssigkeitsundurchlässige rückwärtige Schicht (3) enthält, und mit elastischen Bändern (7), die mittels Befestigungslinien (10A, 10B) auf der Grundanordnung angebracht sind, um eine Taillenöffnung teilweise zu umgeben, wobei die Grundanordnung Umrißlinien aufweist, die jeweils längs verlaufende, gegenüberliegende Seitenränder und quer verlaufende, gegenüberliegende Endränder enthalten, **dadurch gekennzeichnet**, daß jeder der gegenüberliegende Seitenränder mit einem Bund (8) versehen ist, der sich entlang des längsverlaufenden Seitenrands erstreckt und im Zusammenwirken mit dem elastischen Band (7) jeweils eine Beinöffnung umgibt, wobei ein äußerer Seitenrand (13A, 13B) des die Beinöffnung umgebenden Bundes (8) entlang dem zugehörigen gegenüberliegenden Seitenrand der Grundanordnung angebracht ist, und jeweilige Endränder (7A, 7B; 8A, 8B) des die Beinöffnung umgebenden Bundes (8) auf der Grundanordnung mittels jeweiliger Befestigungslinien (11A, 11B) angebracht sind, die an den Umrißlinien des Kleids vorgesehen sind.

2. Hosenartiges Wegwerfkleid nach Anspruch 1, worin die Befestigungslinien (10A, 10B; 11A, 11B) für die jeweiligen Endränder (7A, 7B; 8A, 8B) der die Beinöffnung umgebenden Bünde (8) und des zugehörigen, die Taillenöffnung umgebenden elastischen Bands (7) eine im wesentlichen durchgehende Einzellinie definieren.

3. Hosenartiges Wegwerfkleid nach Anspruch 1 oder 2, worin ein innerer Seitenrand des die Beinöffnung umgebenden Bunds (8) und ein äußerer Seitenrand des die Taillenöffnung umgebenden elastischen Bands (7) miteinander an den Verbindungslinien angrenzen und dazu benachbart sind.

4. Hosenartiges Wegwerfkleid nach einem der Ansprüche 1-3, worin der die Beinöffnung umgebende Bund (8) und das die Taillenöffnung umgebende elastische Band (7) an mindestens einer der Verbindungslinien einander überlappen und dazu benachbart sind, wobei das elastische Band (7) sich zwischen dem die Beinöffnung umgebenden Bund und der Oberschicht (2) befindet.

5. Hosenartiges Wegwerfkleid nach einem der Ansprüche 1-4, worin ein Paar der die Beinöffnung umgebenden Bünde (8) und das die Taillenöffnung umgebende elastische Band durch Bereitstellen eines einzigen Teils mit Schlitz (17) gebildet wird.

6. Hosenartiges Wegwerfkleid nach einem der Ansprüche 1-5, worin das die Beinöffnung umgebende elastische Band (7) mit einem Längsschlitz (23) darin versehen ist, um einen Schulterriemen (35) zu bilden.

## Claims

1. Pants-type disposable garment, comprising a basic component including at least one liquid-permeable upper layer (2) and one liquid-impermeable rear layer (3), and with elastic straps (7) which are attached to the basic component by attachment lines (10A, 10B), in order partially to surround a waist opening, the basic component having contour lines which respectively include longitudinally-extending, oppositely-disposed lateral edges and transversely-extending, oppositely-disposed end edges, characterised in that each of the oppositely-disposed lateral edges is provided with a band (8) which extends along the longitudinally-extending lateral edge and, in co-operation with the elastic strap (7), respectively surrounds a leg opening, an external lateral edge (13A, 13B) of the band (8) surrounding the leg opening being attached to the associated oppositely-disposed lateral edge of the basic component, and respective end edges (7A, 7B; 8A, 8B) of the band (8) surrounding the leg opening are attached to the basic component by means of respective attachment lines (11A, 11B) which are provided on the contour lines of the garment.

2. Pants-type disposable garment according to claim 1, wherein the attachment lines (10A, 10B; 11A, 11B) for the respective end edges (7A, 7B; 8A, 8B) of the bands (8) surrounding the leg opening, and of the associated elastic strap (7) surrounding the waist opening, define a substantially continuous single line.

3. Pants-type disposable garment according to claim 1 or 2,
wherein an internal lateral edge of the band (8) surrounding the leg opening and an external lateral edge of the elastic strap (7) surrounding the waist opening are contiguous with one another at the lines of connection, and are adjacent thereto.

4. Pants-type disposable garment according to one of claims 1 to 3, wherein the band (8) surrounding the leg opening, and the elastic strap (7) surrounding the waist opening, overlap one another at at least one of the lines of connection, and are contiguous therewith, the elastic strip (7) being located between the band surrounding the leg opening and the upper layer (2).

5. Pants-type disposable garment according to one of claims 1 to 4, wherein a pair of the bands (8) surrounding the leg opening, and the elastic strap surrounding the waist opening, is formed by provision of a single part with a slot (17).

6. Pants-type disposable garment according to one of claims 1 to 5, wherein the elastic strap (7) surrounding the leg opening is provided with a longitudinal slot (23) in order to form a shoulder strap (35).

## Revendications

1. Vêtement jetable du type culotte, comprenant une structure de base présentant au moins une couche supérieure (2) perméable aux liquides et une couche arrière (3) imperméable aux liquides, et pourvu de bandes élastiques (7) appliquées sur la structure de base au moyen de lignes de fixation (10A, 10B), pour délimiter, en partie, une ouverture de passage de la taille, ladite structure de base présentant des lignes de contour comportant, respectivement, des bords latéraux longitudinaux opposés, et des bords d'extrémité transversaux opposés, caractérisé en ce que chacun des bords latéraux opposés présente un rabat (8) longeant le bord latéral longitudinal, et délimitant, respectivement, une ouverture de passage des jambes, en combinaison avec la bande élastique (7), vêtement dans lequel un bord latéral extérieur (13A, 13B) du rabat (8) délimitant l'ouverture de passage des jambes, est appliqué le long du bord latéral opposé associé de la structure de base, et où des bords d'extrémité respectifs (7A, 7B ; 8A, 8B) du rabat (8) délimitant l'ouverture de passage des jambes, sont appliqués sur la structure de base au moyen de lignes de fixation (11A, 11B), prévues sur les lignes de contour du vêtement.

2. Vêtement jetable du type culotte selon la revendication 1, dans lequel les lignes de fixation (10A, 10B ; 11A, 11B) prévues pour les bords d'extrémité respectifs (7A, 7B ; 8A, 8B) des rabats (8) délimitant l'ouverture de passage des jambes, et des bandes élastiques (7) associées délimitant l'ouverture de passge de la taille, définissent une ligne individuelle approximativement continue.

3. Vêtement jetable du type culotte selon l'une des revendications 1 ou 2, dans lequel un bord latéral intérieur du rabat (8) délimitant l'ouverture de passage des jambes, et un bord latéral extérieur de la bande élastique (7) délimitant l'ouverture de passage de la taille, se touchent au niveau des lignes de jonction et sont contigus à ces dernières.

4. Vêtement jetable du type culotte selon l'une quelconque des revendications 1-3, dans lequel le rabat (8) délimitant l'ouverture de passage des jambes, et la bande élastique (7) délimitant l'ouverture de passage de la taille, se chevauchent au moins au niveau de l'une des lignes de jonction et sont contigus à cette dernière, ladite bande élastique (7) se trouvant entre le rabat délimitant l'ouverture de passage des jambes et la couche supérieure (2).

5. Vêtement jetable du type culotte selon l'une quelconque des revendications 1-4, dans lequel deux des rabats (8) délimitant les ouvertures de passage des jambes et la bande élastique délimitant l'ouverture de passage de la taille, sont obtenus en pratiquant une fente (17) dans une seule pièce.

6. Vêtement jetable du type culotte selon l'une quelconque des revendications 1-5, dans lequel la bande élastique (7) délimitant l'ouverture de passage de la taille, comporte une fente longitudinale (23), pour former une bretelle (35).
